# FASCICULE DE BREVET EUROPEEN

(11) **EP 0 920 628 B1**
(45) Date de publication et mention de la délivrance du brevet: **05.12.2001**
(21) Numéro de dépôt: 98929487.1
(22) Date de dépôt: 04.06.1998
(51) Int. Cl.: G01N 33/566, G01N 33/86

(54) **NOUVELLE METHODE D'ANALYSE DES RECEPTEURS PLAQUETTAIRES GPIIb/IIIa**
NEUES BESTIMMUNGSVERFAHREN VON BLUTTPLÄTTCHEN-GPIIb/IIIa-REZEPTOREN
NOVEL GPIIb/IIIa PLATELET RECEPTORS ASSAY

(30) Priorité: 06.06.1997 FR 9707059
(43) Date de publication de la demande: 09.06.1999
(73) Titulaire: Biocytex, 13010 Marseille (FR)
(72) Inventeur: BESSON-FAURE, Isabelle, F-13001 Marseille (FR); CANTON, Michel, F-13260 Cassis (FR)
(74) Mandataire: Warcoin, Jacques
(86) Numéro de dépôt international: FR9801135
(87) Numéro de publication internationale: WO9855868

(56) Documents cités:
- WO-A-96/10749
- US-A- 5 470 738
- B. S. COLLER ET AL.: "Rapid and simple platelet function assay to assess glycoprotein IIb/IIIa receptor blockade." CIRCULATION, vol. 95, no. 4, 18 février 1997, pages 860-867, XP002057028
- D. COX ET AL.: "Pentamidine is a specific, non-peptide, GPIIb/IIIa antagonist." THROMBOSIS AND HAEMOSTASIS, vol. 3, no. 75, mars 1996, pages 379-528, XP002057029

## Description

L'invention concerne une méthode d'analyse des récepteurs plaquettaires GPIIb/IIIa occupés par un composé antiagrégant plaquettaire ou par du fibrogène et des récepteurs restés à l'état libre.

Les plaquettes jouent un rôle clef dans l'équilibre de la balance hémostatique. Des signaux activateurs peuvent entraîner à leur niveau des modifications à la fois morphologiques et biochimiques, conjointement à des variations dans les taux d'expression de glycoprotéines de surface. Ces glycoprotéines sont pour la plupart des récepteurs de ligands impliqués dans l'adhésion (ex. le récepteur du facteur von Willebrand : la molécule GPIb), dans l'agrégation (ex. le récepteur du fibrogène : la molécule GPIIb/IIIa) alors que d'autres témoignent de l'état d'activation plaquettaire (ex. la molécule GMP 140 ou P-sélectine).

L'activation des plaquettes et l'agrégation qui en résulte sont des phénomènes physiologiques qui, après dépassement d'un seuil, sont associés à diverses conditions pathologiques telles que les accidents thrombotiques artériels. Ceci explique la raison de l'intérêt de l'industrie pharmaceutique pour le développement de nouvelles molécules thérapeutiques antiagrégantes.

La cible privilégiée dans les nouvelles thérapies développées est le récepteur GPIIb/IIIa qui est spécifiquement exprimé sur les plaquettes. Sous l'effet d'une activation des plaquettes, ce récepteur fixe avec une forte affinité le fibrogène et d'autres protéines d'adhésion, ce qui provoque une agrégation des plaquettes entre elles.

Le récepteur GPIIb/IIIa appartient au groupe des intégrines, composé de différentes molécules impliquées dans les phénomènes d'adhésion cellulaire. Les intégrines sont des hétérodimères α/β et sont classées en plusieurs familles suivant leur sous-unité β. On connaît actuellement une vingtaine d'intégrines dont 9 se lient à leur ligand via la séquence d'acides aminés R-G-D.

La GPIIb/IIIa (α IIbβ3) est un membre de la famille d'intégrines β3. Elle possède la même sous-unité β (mais une sous-unité α différente) que le récepteur de la vitronectine (α vβ3) présent notamment sur les plaquettes et en grande quantité sur les cellules endothéliales. Les sous-unités IIb et IIIa sont classées respectivement dans les groupes CD 41 et CD 61 selon la nomenclature internationale.

Les antiagrégants anti-GPIIb-IIIa plaquettaires appartiennent à deux classes différentes : a) les anticorps monoclonaux antagonistes du fibrinogène, dont le C7E3Fab de l'anticorps monoclonal 7E3 humanisé dirigé contre un épitope du CD 61 et commercialisé sous le nom de RéoPro®, b) les peptides cycliques, type intégréline, qui possèdent la séquence RGD ou KGD, proche de la séquence RGD (Arg-Gly-Asp) commune aux ligands du récepteur plaquettaire, et les peptidomimétiques, type lamifiban ou tirofiban. Ces substances développées actuellement ne sont actives que par voie parentérale. D'autres molécules administrables par voie orale sont en cours de développement. Ces nouvelles molécules peuvent toutefois induire des effets secondaires se traduisant chez les patients traités, par des effets hémorragiques aux points d'accès ou à localisation interne. Pour ces nouvelles molécules qui, par leur nature, restent longtemps en circulation, il n'existe pas actuellement d'antidote. Il est donc important, dans le cadre d'une thérapie antiplaquettaire, non seulement de pouvoir adapter la dose thérapeutique d'agent administré, mais également de disposer rapidement durant le suivi du traitement, d'informations sur le nombre de récepteurs GPIIb/IIIa occupés par l'antagoniste, de façon à pouvoir contrôler les complications hémorragiques tout en conservant une quantité suffisante d'antagoniste fixé pour obtenir un effet antiagrégant.

C'est pourquoi la présente invention concerne un procédé de dosage sur un échantillon à tester de l'occupation du récepteur GPIIb/IIIa par un antagoniste du fibrinogène, caractérisé en ce qu'on dose le nombre de récepteurs occupés à l'aide d'un anticorps **[Mab1]** compétiteur de l'antagoniste ainsi que le nombre de récepteurs totaux occupés ou non à l'aide d'un anticorps **[Mab2]** dit non compétiteur, spécifique du récepteur occupé ou non, on en déduit l'occupation des récepteurs GPIIb/IIIa dans l'échantillon.

Par occupation on entend désigner aussi bien la quantification en nombre absolu des récepteurs plaquettaires du fibrinogéne GPIIb/IIIa occupés par un antiagrégant par rapport au nombre total de récepteurs dans un échantillon sanguin qu'un rapport dont la variation peut être étudiée dans le temps.

Il est également possible de déterminer le nombre de récepteurs totaux avant toute médication sans avoir à répéter ce dosage dans le cadre du suivi du traitement.

De même, lorsqu'il est précisé que l'on dose le nombre de récepteurs, il peut s'agir d'un dosage seulement semi-quantitatif.

Bien entendu, lorsque l'on souhaite mesurer précisément le nombre de récepteurs, il est nécessaire de prévoir des gammes d'étalonnage avec des courbes d'étalonnage.

Les anticorps anti-GPIIb/IIIa utilisés dans le cadre de la présente invention sont de préférence dirigés contre un épitope de la sous-unité GPIIIa (CD 61).

Il s'agit plus particulièrement des anticorps SZ 21 et P2 qui sont commercialisés par la société Immunotech et l'anticorps PM 6/13 qui est commercialisé par la société Cymbus.

Il peut s'agir également de l'anticorps monoclonal P18 déposé à la Belgian Coordinated Collection of Microorganisms (BCCM) sous le numéro LMBP 1662CB le 5 juin 1997 (ce dépôt n'est accessible qu'aux experts, conformément aux dispositions légales).

L'anticorps PM 6/13 est disponible chez Cymbus Bioservices Limited (UK), c'est un anti-CD 61 de type IgG1 SZ 21 disponible chez Immunotech (France) et un anti-CD 61 de type IgG1, quand à P2, disponible chez la même société, il s'agit d'un IgG1 anti-CD 41.

Les couples d'anticorps utilisés dépendront principalement du type d'antiagrégant utilisé. En effet, le RéoPro et les peptides de type RGD sont des antiagrégants qui se fixent sur des sites différents de la GPIIIa.

Le RéoPro présente un site proche de celui du fibrinogène et inhibe sa fixation par encombrement stérique, au contraire les peptides RGD ont le même site que le fibrinogène, dans une compétition directe pour la séquence RGD.

De ce fait, le produit le plus adapté pour le dosage des récepteurs occupés par du RéoPro est le couple anticorps compétiteur P2 d'Immunotech et anticorps non compétiteur PM 6/13 de Cymbus, alors que dans le cas des peptides RGD, l'anticorps compétiteur est l'anticorps PM 6/13 de Cymbus et l'anticorps non compétiteur est l'anticorps SZ 21 d'Immunotech. En effet, l'anticorps PM 6/13 est non compétiteur lorsque l'antiagrégant est le RéoPro, mais devient compétiteur s'il s'agit d'un peptide RGD.

Le dosage mettant en oeuvre les anticorps monoclonaux précédents est de préférence un dosage par cytométrie quantitative mais on peut envisager d'autres méthodes inspirées notamment de l'Elisa ou un autre type de procédé.

De préférence le dosage est effectué par révélation des anticorps spécifiques à l'aide d'anticorps marqués par un fluorochrome en utilisant en particulier la méthode d'immuno-fluorescence indirecte pouvant être associée à une méthode quantitative.

La méthode dite cytométrie de même que la méthode quantitative d'immuno-fluorescence indirecte (dite méthode QIFI) sont connues et ne seront pas redécrites en détail (P. Poncelet et al., 1985, J. Immunol. Methods 85, 65-74).

L'échantillon utilisé est de préférence un échantillon biologique, notamment échantillon sanguin, échantillon de sang total prélevé sur citrate de sodium ou citrate théophilline adénosine dipyrridanol (CTAD) (inhibiteur des fonctions plaquettaires), ou bien fraction riche en plaquettes, plasma riche en plaquettes (PRP) ou plaquettes fixées par exemple.

Bien qu'on ait mentionné précédemment des couples d'anticorps spécifiques, il est possible d'utiliser d'autres anticorps que l'on pourra notamment sélectionner dans les listes figurant dans l'ouvrage intitulé « Leucocyte Typing V, White Cell Differentiation Antigens ; Proceedings of the Fifth International Workshop and Conference Held in Boston,USA, 3-7 novembre 1993, volume 2, Oxford University Press, 1995 », dans la mesure où pour les anticorps anti-GPIIb/IIIa compétiteurs, on sélectionne un anticorps dirigé contre un épitope du complexe GPIIb/IIIa dont le taux de fixation à celui-ci chute d'au moins 50% et de préférence 75% en présence d'antiagrégant plaquettaire utilisé à concentration saturante, mais dont la fixation ne doit pas inhiber celle de l'antiagrégant aux plaquettes.

Pour ce qui concerne l'anticorps anti-GPIIb/IIIa non compétiteur, il s'agira d'un anticorps dirigé contre un épitope du couple GPIIb/IIIa dont le taux de fixation à celui-ci ne varie pas plus de 15% en présence et en absence d'antiagrégant plaquettaire.

La présente invention concerne également une trousse de dosage destinée à la mise en oeuvre du procédé précédent, caractérisée en ce qu'elle comprend au moins :
- un anticorps monoclonal du récepteur GPIIb/IIIa compétiteur d'un antagoniste du fibrinogène et,
- un anticorps monoclonal spécifique du récepteur GPIIb/IIIa occupé ou non.

On peut rappeler ci-après, brièvement le principe de quantification des antigènes de surface tels que les récepteurs GPIIb/IIIa.

Les sites antigéniques cellulaires à quantifier sont marqués en immuno-fluorescence indirecte, avec fixation d'un Mab préférentiellement de type IgG et notamment de souris, spécifique de l'antigène à étudier, révélé par un polyclonal anti-IgG de souris conjugué à un fluorochrome. L'intensité de fluorescence obtenue en cytométrie de flux est une unité arbitraire qui est cependant étroitement liée au niveau d'expression cellulaire de l'antigène. La standardisation des données cytométriques est assurée par l'introduction, au cours de chaque immuno-marquage d'un calibrant traité en parallèle aux tubes échantillons. Ce calibrant est constitué de billes de latex recouvertes de quantités croissantes et définies de Mab de type IgG. L'analyse de ce calibrant permet la réalisation d'une gamme d'étalonnage reliant les intensités de fluorescence au nombre absolu d'antigène par cellule.

Les exemples ci-après permettent de mettre en évidence les caractéristiques et avantages de la présente invention.

Le test proposé est réalisé sur un échantillon de sang total prélevé sur citrate de sodium (ou CTAD). Sur le modèle du CYTOQUANT Gp la technique de marquage est effectuée directement sur le sang total et ne nécessite pas d'étape de lavage. Pour ce test, trois paramètres sont étudiés par échantillon (3 tubes) : le sang est mis en présence d'un Mab témoin négatif (marquage non spécifique), d'un Mab Mab1 et d'un Mab **Mab2.** La gamme d'étalonnage est ajoutée dans un tube lors de la seconde étape du marquage, en présence de l'Ac de révélation fluorescent. L'intensité de fluorescence due à l'immuno-marquage est ensuite lue au cytomètre. Cette intensité de fluorescence est proportionnelle au nombre de Mab spécifique fixé sur les plaquettes. La transformation de l'intensité de fluorescence en nombre absolu de Mab fixé par plaquette est réalisée après construction de la droite d'étalonnage.

Les résultats sont exprimés de la façon suivante :
- nombre total de molécules de GPIIb/IIIa par plaquette (**valeur Mab2**)
- nombre de molécules GPIIb/IIIa occupés par la molécule anti-agrégante **(différence Mab2-Mab1).**

Ainsi, deux paramètres peuvent être étudiés dans le même temps.

### EXEMPLE 1

Lors de la phase de faisabilité, différents Mabx spécifiques de la GPIIb/IIIa ont été testés sur plaquettes en présence et en l'absence de ReoPro.

### Détermination de la concentration saturante du ReoPro sur plaquette à l'état basal et après activation à l'ADP.

### Protocole 1

Test en immuno-fluorescence indirecte en remplaçant le Mab spécifique par du ReoPro a différentes dilutions.

### 1- Matériel

- ReoPro 2 mg/ml, dilutions en PBS-BSA 0,1% de 50 µg/ml → 0,75 µg/ml
- ADP Stago 0,2 µM
- DDAF

### 2 - Méthode

- Mélanger vol/vol PRP/dilutions de ReoPro (mél 1)
   PRP/PBS tampon (mél 2)
   Incubation 15 mn TA
- Mélanger vol/vol mélange 1/PBS mélange 1/ADP 0,2 µM
   mélange 2/PBS
   mélange 2/ADP 0,2 µM
   Incubation 5 mn TA
- Dans chacun des 'mélanges, ajouter 4 ml PBS BA. Centrifugation 3000t/mn 10 mn. Eliminer le surnageant.
- Culot + 100µl DDAF (réactif mouton anti souris - FITC) Incubation 15 mn TA
- + 2 ml PBS → Analyse CMF
N.B. Il existe autant de tubes mél 1 que de dilutions ReoPro.

Le ReoPro est un anticorps de type F(ab) chimère avec une partie immuno-réactive Fv de souris associée à une partie constante d'IgG humaine. Le second réactif, polyclonal de mouton anti Ig de souris conjugué FITC, se fixe faiblement sur la molécule ReoPro mais suffisamment pour obtenir un signal analysable. La concentration saturante se situe à partir de 1µg/ml concentration finale. L'activation plaquettaire à l'ADP ne modifie pas la fixation du ReoPro.

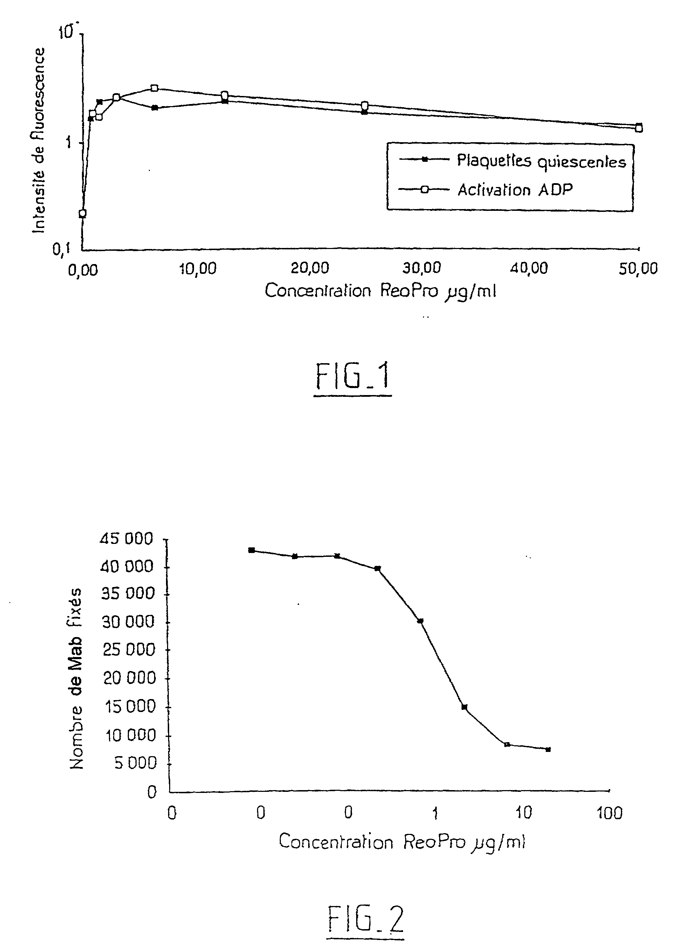

Les résultats du test sont reportés sur la figure 1.

### Recherche de Mabx d'une part complémentaires et d'autre part compétiteurs de la fixation du ReoPro

### Protocole 2

### 1- Matériel

- ReoPro 2 mg/ml dilué à 10 µg/ml en PBS BSA 0,1%
- Mabx CD41 et CD61 (anti GPIIb/IIIa et anti GPIIIa) à 10 µg/ml.
- DDAF (mouton anti Ig de souris FITC).

### 2 - Méthode

- Mélanger vol/vol PRP/ReoPro 10 µg/ml
   PRP/PBS BSA 0,1%
   Incubation 5 mn TA
- Lavage des 2 mélanges (+4 ml PBS BSA 0,1%, centrifugation 3000 t/mn 10 mn, éliminer le surnageant).
   Culot + PBS BSA (vol = vol de PRP initial)
- 20 µl de chaque mélange + 20 µl de chaque Mab à tester.
   Incubation 10 mn TA
- + 100 µl DDAF 1/100 Incubation 10 mn TA
- + 2 ml PBS BSA 0,1% --> analyse CMF

Nous avons testé 9 Mabx spécifiques de la GPIIb/IIIa (CD41 et CD61) (voir tableau 1).

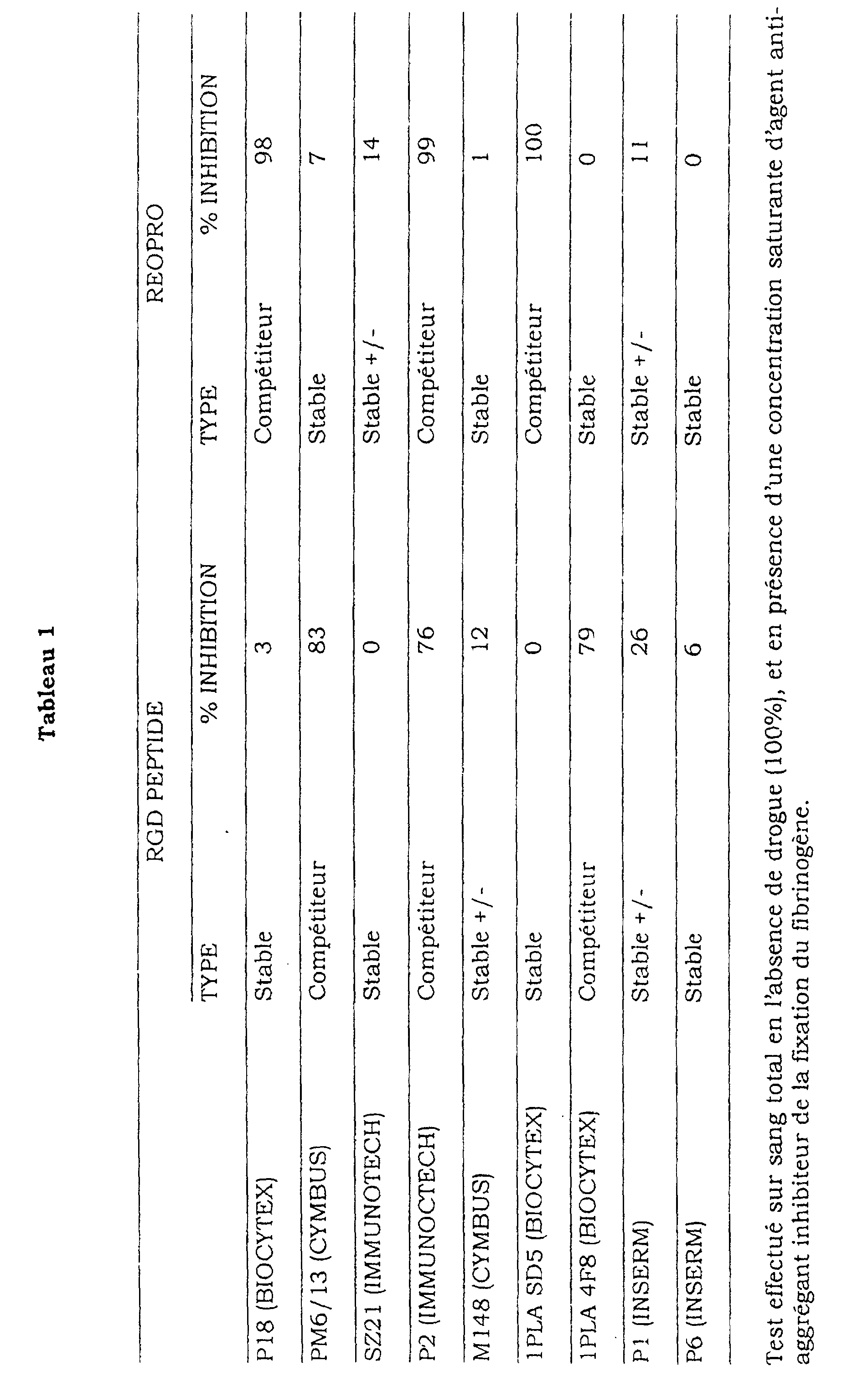

Nous avons comparé les valeurs quantitatives obtenues sur plaquettes pré-incubées en présence de Reopro (10 µg/ml final) ou de PBS. Nous avons choisi le Mab PM6/13 dont l'expression ne varie pas de plus de 10% en présence et en l'absence de ReoPro et le Mab P18 dont l'expression chute de + de 95% en présence de ReoPro utilisé à concentration saturante.

Le tableau 1 montre les résultats obtenus avec les mêmes anticorps testés sur du sang total.

L'intérêt d'utiliser le couple PM16/13-P18 réside dans la réponse comparable observée sur plaquette pour les 2 Mabx en l'absence d'antiagrégant.

Lorsqu'on fait varier la concentration de pré-incubation du ReoPro, en expérience dose/réponse avec le protocole 3, la fixation du P18 est inversement proportionnelle à celle de l'anti-agrégant comme indiqué dans la figure 2.

### Conclusion

- Spécificité :: Mab1 clone P18 (CD61, anti GPIII a) Mab2 clone PM6/13 (CD61, anti GPIIIa)
- Source :: Clone P18 (Mab IgG2a) INSERM Clone PM6/13 (Mab IgG1) Cymbus

Un réactif (Fab)'2 mouton anti Ig totales de souris - FITC, le DDAF constitue le réactif polyclonal le plus approprié.

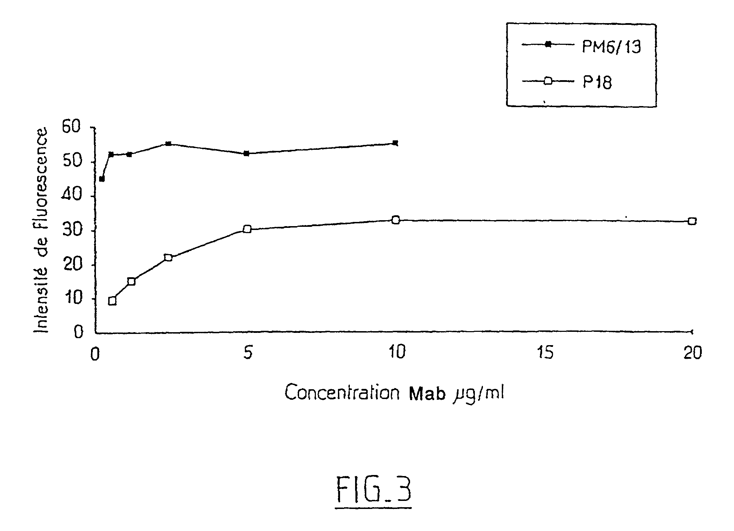

La figure 3 est une étude de saturation sur plaquettes des Mabx CD 61 P18 et PM 6/13 purifiés.

### Protocole 3

### 1- Matériel

- ReoPro 2 mg/ml dilutions en PBS BSA 0,1%, de 3 en 3 de 200 µg/ml à 0,09 µg/ml
- Mab P18 : Ascite 1/800
- Mab PM6/13 : 10 µg/ml
- DDAf
- PRP

### 2- Méthode

- Dilution 1/10 de chaque préparation de ReoPro en PRP.
   Incubation 5 mn TA
- Lavage (en PBS BSA 0,1% 3000 t/mn 10 mn)
- Culot + PBS BSA 0,1% (vol égal au vol de PRP de départ)
- 20 µl de préparation plaquettaire + 20 µl du Mab P18, PM6/13 et IgG, témoin négatif.
   Incubation 10 mn TA
- + 100 µl DDAF 1/100 sur chaque immuno-marquage et sur 40 µl de gamme de calibration de type CYTOQUANT GP.
   Incubation 10 mn TA
- + 2 ml PBS BSA 0,1% → CMF.

### EXEMPLE 2

Des dosages équivalents ont été réalisés en utilisant :
PM6/13 comme Mab2 et,
   P18 comme Mab1
dans un modèle in vitro sur du sang total avec comme antiaggrégant le ReoPro à différentes concentrations.

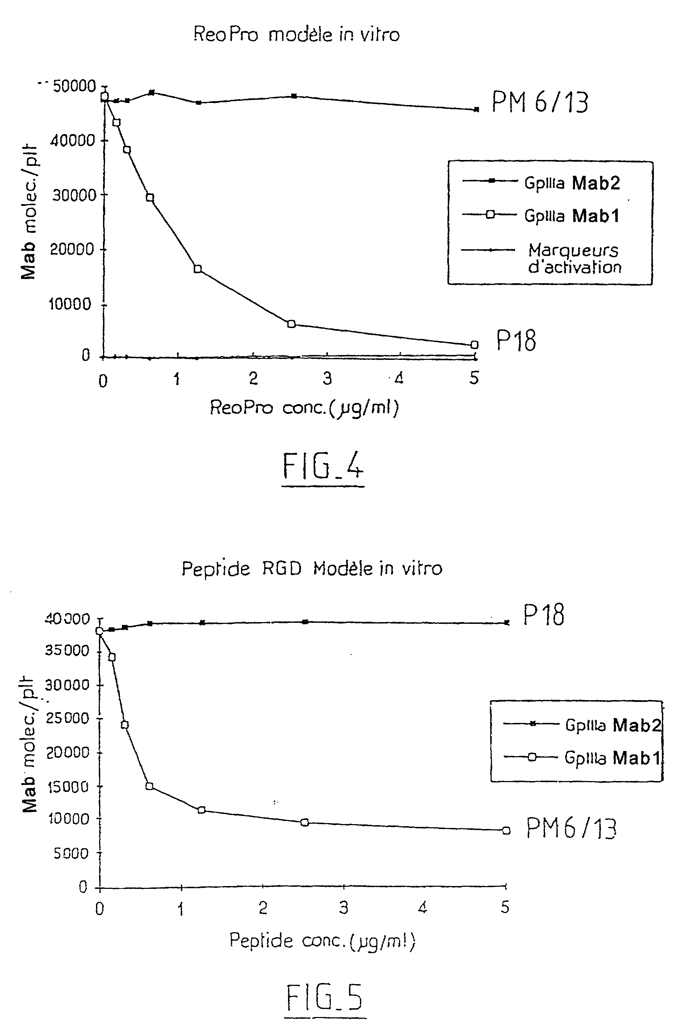

Les résultats sont rassemblés sur la figure 4.

### EXEMPLE 3

Des dosages équivalents ont été réalisés en utilisant :
P18 comme Mab2
PM6/13 comme Mab1
dans un modèle in vitro utilisant comme antiaggrégant le peptide RGD à différentes concentrations.

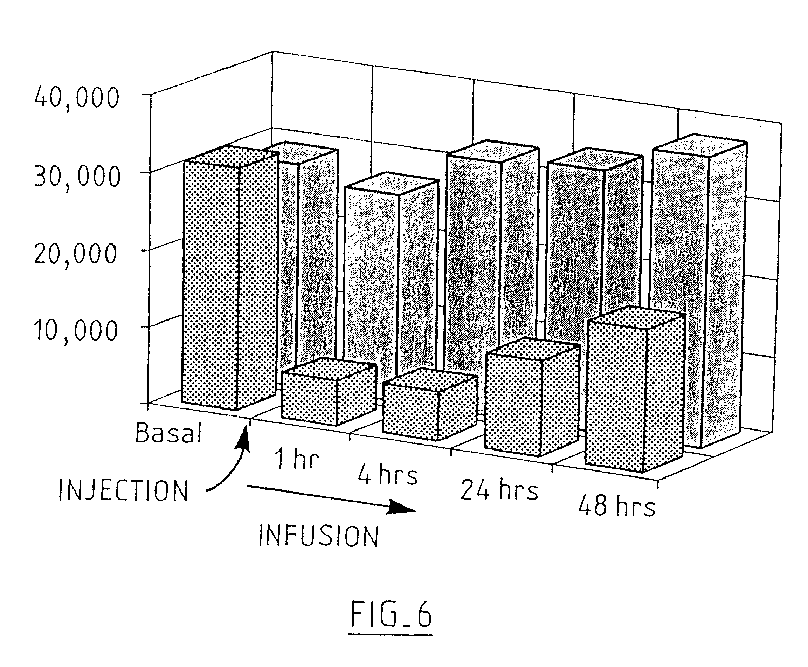

Les résultats sont rassemblés sur la figure 5.

Dans les exemples 2 et 3, la drogue utilisée (Réopro ou peptide RGD) est diluée en tampon phosphate pour obtenir une série de dilutions de 1 à 50 µg/ml. 5 µl sont mélangés à 45 µl de sang total (dilution 1/10). On incube à température ambiante 30' puis on ajoute 150 µl de tampon.

Sur des aliquots de 20 µl de sang total dilué on rajoute 20 µl de Mab testé à 10 ou 20 µg/ml.

Après incubation et addition d'Ac anti-Ig de souris conjugué au FITC, on effectue une analyse cytométrique.

Le nombre de molécules de Mabx fixées par plaquettes est calculé en utilisant une droite de calibration tracée à partir de billes de latex, conformément à la méthode QIFI.

### EXEMPLE 4

Des essais ont été réalisés in vivo sur des patients traités au ReoPro.

Dans ce cas, on ne rajoute pas de RéoPro. On prélève du sang total (20 µl dilué au 1/4 dans un tampon physiologique).

On ajoute 20 µl de Mab (P18 ou PM 6/13) à 10 µg/ml. On incube 10' à température ambiante (TA). On ajoute ensuite 20 µl de réactif et on effectue une lecture cytométrique après incubation (10' TA) et dilution appropriée.

La figure 6 montre le nombre de récepteurs libres après l'injection par rapport au nombre total de récepteurs. Au bout de 48 heures, le nombre de récepteurs libres est encore inférieur à 50%.

Les Mab utilisés sont PM6/13 pour le dosage des récepteurs totaux et P18 pour le dosage des récepteurs libres.

## Revendications

1. Procédé de dosage sur un échantillon à tester de l'occupation du récepteur cellulaire GPIIb/IIIa par un antagoniste du fibrinogène, **caractérisé en ce qu'**on dose dans ledit échantillon le nombre de récepteurs occupés à l'aide d'un anticorps compétiteur Mab1 de l'antagoniste ainsi que le nombre de récepteurs totaux occupés ou non à l'aide d'un anticorps spécifique du récepteur occupé ou non, Mab2 dit non-compétiteur et on en déduit le taux d'occupation des récepteurs GPIIb/IIIa dans l'échantillon.

2. Procédé selon la revendication 1, **caractérisé en ce que** le nombre de récepteurs est mesuré par comparaison avec des courbes d'étalonnage.

3. Procédé selon l'une des revendications 1 à 2, **caractérisé en ce que** les anticorps sont dirigés contre GPIIIa.

4. Procédé selon l'une des revendications 1 à 3, **caractérisé en ce que** le dosage est effectué par marquage des anticorps à l'aide d'un anticorps marqué.

5. Procédé selon la revendication 4, **caractérisé en ce que** l'anticorps de marquage est marqué par un fluorochrome.

6. Procédé selon l'une des revendications 1 à 5, **caractérisé en ce que** le dosage est effectué par cytométrie quantitative.

7. Procédé selon l'une des revendications 1 à 6, **caractérisé en ce que** le dosage est effectué par la méthode quantitative d'immuno-fluorescence indirecte.

8. Procédé selon l'une des revendications 1 à 7, **caractérisé en ce que** Mab1 est l'anticorps monoclonal PM6/13 et Mab2 est l'anticorps monoclonal SZ 21 ou P18 produit par l'hybridome déposé sous le numéro d'accession LMBP 1662 CB, dans le cas où les antagonistes sont des peptides RGD.

9. Procédé selon l'une des revendications 1 à 7, **caractérisé en ce que** Mab1 est l'anticorps monoclonal P2 ou P18 produit par l'hybridome déposé sous le numéro d'accession LMBP 1662 CB et Mab2 est l'anticorps monoclonal PM6/13 dans le cas où l'antiagrégant est l'anticorps 7E3 ou ses fragments.

10. Procédé selon l'une des revendications 1 à 9, **caractérisé en ce que** l'échantillon à tester est un échantillon sanguin.

11. Procédé selon la revendication 10, **caractérisé en ce que** l'échantillon à tester est un échantillon enrichi en plaquettes.

12. Trousse de dosage destinée à la mise en oeuvre du procédé selon l'une des revendications 1 à 11, **caractérisée en ce qu'**elle comprend au moins :
- un anticorps monoclonal du récepteur GPIIb/IIIa compétiteur d'un antagoniste du fibrinogène et,
- un anticorps monoclonal spécifique du récepteur GPIIb/IIIa occupé ou non.

## Claims

1. Method of determining, on a test sample, the occupation of the cellular GPIIb/IIIa receptor by an antagonist of fibrinogen, **characterized in that** the number of receptors occupied is determined in the said sample with the aid of an antibody Mab1 which is a competitor for the antagonist, and the number of occupied or unoccupied total receptors is determined with the aid of a so-called noncompetitor antibody Mab2 specific for the occupied or unoccupied receptor and the rate of occupation of the GPIIb/IIIa receptors in the sample is deduced therefrom.

2. Method according to Claim 1, **characterized in that** the number of receptors is measured by comparison with calibration curves.

3. Method according to either of Claims 1 and 2, **characterized in that** the antibodies are directed against GPIIIa.

4. Method according to one of Claims 1 to 3, **characterized in that** the determination is carried out by labelling the antibodies with the aid of a labelled antibody.

5. Method according to Claim 4, **characterized in that** the antibody is labelled with a fluorochrome.

6. Method according to one of Claims 1 to 5, **characterized in that** the determination is carried out by quantitative cytometry.

7. Method according to one of Claims 1 to 6, **characterized in that** the determination is carried out by the indirect quantitative immunofluorescence method.

8. Method according to one of Claims 1 to 7, **characterized in that** Mab1 is the monoclonal antibody PM6/13 and Mab2 is the monoclonal antibody SZ21 or P18 produced by the hybridoma deposited under the accession number LMBP 1662CB.

9. Method according to one of Claims 1 to 7, **characterized in that** Mab1 is the monoclonal antibody P2 or P18 produced by the hybridoma deposited under the accession number LMBP 1662CB and Mab2 is the monoclonal antibody PM6/13 in the case where the anti-aggregation agent is the antibody 7E3 or fragments thereof.

10. Method according to one of Claims 1 to 9, **characterized in that** the sample to be tested is a blood sample.

11. Method according to Claim 10, **characterized in that** the sample to be tested is a sample enriched in platelets.

12. Assay kit intended for carrying out the method according to one of Claims 1 to 11, **characterized in that** it comprises at least:
- one monoclonal antibody for the GPIIb/IIIa receptor which is a competitor for a fibrinogen antagonist, and
- one monoclonal antibody specific for the occupied or unoccupied GPIIb/IIIa receptor.

## Patentansprüche

1. Verfahren zur Mengenbestimmung des besetzten zellulären GPIIb/IIIa-Rezeptors durch einen Fibrinogenantagonisten in einer zu testenden Probe, **dadurch gekennzeichnet, dass** man in der Probe die Anzahl der besetzten Rezeptoren mit Hilfe eines Antikörperkompetitors, Mab1, des Antagonisten sowie die Anzahl der insgesamt besetzten oder unbesetzten Rezeptoren mit Hilfe eines spezifischen Antikörpers des besetzten oder unbesetzten Rezeptors, Mab2, genannt Nicht-Kompetitor, bestimmt und daraus den Anteil an besetzten GPIIb/IIIa-Rezeptoren in der Probe bestimmt.

2. Verfahren gemäß Anspruch 1, **dadurch gekennzeichnet, dass** die Anzahl der Rezeptoren durch Vergleich mit den Eichkurven bestimmt wird.

3. Verfahren gemäß wenigstens einem der Ansprüche 1 und 2, **dadurch gekennzeichnet, dass** die Antikörper gegen GPIIIa gerichtet sind.

4. Verfahren gemäß wenigstens einem der Ansprüche 1 bis 3, **dadurch gekennzeichnet, dass** die Mengenbestimmung durch Markierung der Antikörper mit Hilfe eines markierten Antikörpers erreicht wird.

5. Verfahren gemäß Anspruch 4, **dadurch gekennzeichnet, dass** der Markierungsantikörper mit Fluorochrom markiert ist.

6. Verfahren gemäß wenigstens einem der Ansprüche 1 bis 5, **dadurch gekennzeichnet, dass** die Mengenbestimmung durch quantitative Cytometrie erreicht wird.

7. Verfahren gemäß wenigstens einem der Ansprüche 1 bis 6, **dadurch gekennzeichnet, dass** die Mengenbestimmung durch die quantitative Methode der indirekten Immunofluoreszenz erreicht wird.

8. Verfahren gemäß wenigstens einem der Ansprüche 1 bis 7, **dadurch gekennzeichnet, dass**, wenn die Antagonisten RGD-Peptide sind, Mab1 der monoklonale Antikörper PM6/13 und Mab2 der monoklonale Antikörper SZ21 oder P18, welcher von dem unter der Nummer LMBP 1662CB hinterlegten Hybridom produziert wird, sind.

9. Verfahren gemäß wenigstens einem der Ansprüche 1 bis 7, **dadurch gekennzeichnet, dass**, wenn der Antiagregant der Antikörper 7E3 oder eines seiner Fragmente ist, Mab1 der monoklonale Antikörper P2 oder P18, welcher von dem unter der Nummer LMBP 1662CB hinterlegten Hybridom produziert wird, und Mab2 der monoklonale Antikörper PM6/13 sind.

10. Verfahren gemäß wenigstens einem der Ansprüche 1 bis 9, **dadurch gekennzeichnet, dass** die zu testende Probe eine Blutprobe ist.

11. Verfahren gemäß Anspruch 10, **dadurch gekennzeichnet, dass** die zu testende Probe eine mit Blutplättchen angereicherte Probe ist.

12. Bestimmungssystem, welches zur Anwendung des Verfahrens gemäß einem der Ansprüche 1 bis 11 bestimmt ist, **dadurch gekennzeichnet, dass** es zumindest
- einen monoklonalen Antikörper des GPIIb/IIIaRezeptors, Kompetitor eines Fibrogenantagonisten, und
- einen spezifischen monoklonalen Antikörper des besetzten oder unbesetzten GPIIb/IIIa-Rezeptors enthält.
